# EUROPEAN PATENT APPLICATION

(11) **EP 2 335 711 A2**
(43) Date of publication of application: **22.06.2011**
(21) Application number: 09749353.0
(22) Date of filing: 19.05.2009
(51) Int. Cl.: A61K 36/24, A61K 31/047, A61K 31/7048

(54) **EXTRACT OF HANCORNIA SPECIOSA AND PHARMACEUTICAL COMPOSITION THEREOF**

(30) Priority: 19.05.2008 BR PI0802004
(71) Applicant: Universidade Federal de Minas Gerais-UFMG, CEP-31270-901 Belo Horizonte, MG (BR)
(72) Inventor: CASTRO BRAGA, Fernão, Cep: 31270-020 (BR); DE FRANÇA CÔRTES, Steyner, Cep: 31270-215 (BR); CAROLINE DA SILVA, Grazielle, Cep: 30570-690 (BR); COUTINHO ENDRINGER, Denise, Cep: 29102-200 (BR)
(74) Representative: Kaiser, Jürgen
(86) International application number: PCT/BR2009/000142
(87) International publication number: WO 2009/140749

(57) **Abstract**

The present invention describes a standardized extract obtained from the leaves of *Hancornia speciosa* Gomes (EHS), popularly known as "mangaba" in Brazil, as well as a standardized fraction with inhibiting activity on the angiotensin converting enzyme (ACE), and vasodilating, anti-hypertensive and antioxidant properties. The invention further relates to the preparation of pharmaceutical compositions that contain said extract or fractions derived from the extract of *Hancornia speciosa Gomes* leaves rich in cyclitols and flavonoids, as well as the use of same for the treatment of cardiovascular diseases such as arterial hypertension, atherosclerosis, stenosis, and non-limiting cardiac or cerebral ischaemia.

## Description

The present invention describes a standardized extract obtained from the leaves of *Hancornia speciosa* Gomes (EHS), popularly known as "mangaba" in Brazil, as well as a fraction enriched in bornesitol, rutin and quinic acid, whose activities can be considered as an angiotensin converting enzyme (hereinafter ACE) inhibitor, vasodilating, anti-hypertensive and antioxidant. Particularly, the present invention refers to obtaining a fraction enriched in the enantiomer L-(+)-bornesitol, rutin and quinic acid from the leaves of the aforesaid vegetal species containing activities that are ACE inhibiting, vasodilating, anti-hypertensive and antioxidant.

This invention also comprises pharmaceutical preparations containing extracts or fractions derived from the species *Hancornia speciosa* Gomes, which are enriched in cyclitols and flavonoids, as well as the use of the mentioned preparations for treating cardiovascular diseases, such as, though nonlimiting, arterial hypertension, atherosclerosis, restenosis, and cardiac or cerebral ischemia.

The fractions and substances presented herein have been assayed and have been showed to possess activities that are ACE inhibiting, vasodilating dependent and independent of the vascular endothelium, hypotensive and antioxidant. The aforesaid fractions and substances have also showed to be able to reduce arterial pressure in hypertensive animal models.

### The State of the Art:

Historically, medicinal plants constitute an effective source for discovering and developing pharmaceutical products used in modern therapies (World Health Organization. Traditional Medicine Strategy 2002-2005. Geneva: World Health Organization, 2002. 74 p.; NEWMAN, D. J.; CRAGG, G. M.; SNADER, K. M. Natural Products as Sources of New Drugs over the Period 1981-2002. J. Nat. Prod., v. 66, p. 1022 - 1037, 2003; BUTLER, M. S. Natural products to drugs: natural product derived compounds in clinical trials. Nat. Prod. Rep., v. 22, p. 162 - 195, 2005; CHIN, Y. W.; BALUNAS M. J.; CHAI, H. B.; KINGHORN, A. D. Drug Discovery From Natural Sources. AAPS J., v. 8, n. 2, p. E23- E253, 2006). The total of 122 natural products from plants are used as drugs nowadays, 80% of which are related to ethnopharmacological use (FABRICANT, D. S.; FARNSWORTH, N. R. The value of plants used in traditional medicine for drug discovery. Environ. Health Perspect., v. 109, p. 6-75, 2001).

In the period between 1940-2002, 140 drugs were approved for cancer treatment, among them 20 (14.3%) were natural products, 31 (22.1%) were derived from natural products, 28 (20%) were products from total or partial syntheses based on natural prototypes, and 41 (29.3%) were products based on random synthesis. In the case of antihypertensive drugs, from the 75 new drugs that were approved between 1981 and 2002, 1 (1.3%) was derived from natural product, 48 (60.8%) were synthesized based on natural prototypes, and 26 (32.9%) were products based on random syntheses (NEWMAN, D. J.; CRAGG, G. M.; SNADER, K. M. Natural Products as Sources of New Drugs over the Period 1981-2002. J. Nat. Prod., v. 66, p. 1022 - 1037, 2003). Despite the fact that the number of new derived compounds from natural products was reduced in 2004, several such pharmaceuticals have been registered for Phase III clinical trials in the United States in that very year (BUTLER, M. S. Natural products to drugsnatural product derived compounds in clinical trials. Nat. Prod. Rep., v. 22, p. 162 - 195, 2005). Therefore, natural products can be asserted as a promising source for the discovery of new pharmaceutical drugs.

Brazil possesses a rich biodiversity, which is distributed among different biomes, including *Mata Atlântica* (Atlantic Tropical Rainforest), *Floresta Amazônica* (Amazon Tropical Rainforest), *Caatinga* (Tropical Scrub Forest), *Pantanal* (Temperate Flooded Grassland), *Floresta de Araucária* (Brazilian Pine Forest), and *Cerrado* (Tropical Grassland and Savannah). The latter is located in the Brazilian central plateau region, comprising the states of Minas Gerais, Mato Grosso, Mato Grosso do Sul, Goiás, Tocantins, São Paulo, Paraná, Maranhão and Piauí, and is the second largest Brazilian biome with approximately 2x10⁶ Km². The Cerrado possesses a herbaceous vegetation with scattered trees and bushes. Its rich flora comprises thousands of vascular plant species belonging to hundreds of genera and families. Several cerrado plants are tradicionally used for medicinal purposes. Cerrado is therefore a potential source of bioactive phytochemicals (FERREIRA, M. B. Plantas portadoras de substâncias medicamentosas, de uso popular, nos cerrados de Minas Gerais. Inf. Agropec., v. 6, n. 61, p. 1- 23, 1980; GOTTSBERGER, I. S. O cerrado como potencial de plantas medicinais e tóxicas. Oréades, v. 8 (14/15), p. 15 - 30, 1981/1982; HIRSCHMANN, G. S.; ARIAS, A. R. A survey of medicinal plants of Minas Gerais, Brazil. J. Ethnopharmacol., v. 29, p. 15- 172, 1990; GAVILANES, M. L.; BRANDO, M. Frutos, folhas e raízes de plantas do cerrado, suas propriedades medicinais, tendo como veículo a cachaça. Inf. Agropec., v. 16, p. 4- 44, 1992; KREG, T.; FIGUEIREDO, I. B.; SANO, E. E.; ALMEIDA, C. A.; SANTOS, J. R.; MIRANDA, H. S.; SATO, M. N.; ANDRADE, S. M. S. Greenhouse gas emissions from biomassburning in the non-anthropogenic cerrado using orbital data. Brasilia: Brazilian Ministry of Science and Technology, Secretariat of Science and Technology Policies and Programs, Department of Thematic Programs, 2002).

Few species occur in the whole extension of the Cerrado; *Hancornia speciosa* Gomes (Apocynaceae) is one of these (GOTTSBERGER, I. S. O cerrado como potencial de plantas medicinais e tóxicas. Oréades, v. 8 (14/15), p. 15 - 30, 1981/1982; SILVA-FILHO, P. V.; BRANDO, M. Plantas medicinais de uso popular coletadas e comercializadas na região metropolitana de Belo Horizonte. Daphne, v. 2, n. 2, p. 3- 53, 1992). The species is populary recommended for treating several diseases, according to ethnobotanic surveys carried out in cerrado. Its barks are used for dermatosis (FERREIRA, M. B. Plantas portadoras de substâncias medicamentosas, de uso popular, nos cerrados de Minas Gerais. Inf. Agropec., v. 6, n. 61, p. 1- 23, 1980; BARROS, M. A. G. E. Plantas medicinais - usos e tradições em Braslia- DF. Oréades, v. 8, n. 14/15, p. 14- 151, 1981/1982, BRITTO, K. B.; BRITTO, I. C. Plantas com atributos medicinais do Herbário da Universidade de Feira de Santana. Oréades, v. 8, n. 14/15, p. 152 - 163, 1981/1982; SILVA-FILHO, P. V.; BRANDO, M. Plantas medicinais de uso popular coletadas e comercializadas na região metropolitana de Belo Horizonte. Daphne, v. 2, n. 2, p. 3- 53, 1992; RODRIGUES, V. E. G.; CARVALHO, D. A. Levantamento etnobotânico de plantas medicinais no domínio Cerrado na região do Alto Rio Grande - Minas Gerais. Cienc. Agrotec., v. 25, n. 1, p. 102 - 123, 2001), to treat liver diseases (FERREIRA, M. B. Plantas portadoras de substâncias medicamentosas, de uso popular, nos cerrados de Minas Gerais. Inf. Agropec., v. 6, n. 61, p. 1- 23, 1980; BARROS, M. A. G. E. Plantas medicinais - usos e tradições em Braslia- DF. Oréades, v. 8, n. 14/15, p. 14- 151, 1981/1982; BRITTO, K. B.; BRITTO, I. C. Plantas com atributos medicinais do Herbário da Universidade de Feira de Santana. Oréades, v. 8, n. 14/15, p. 152 - 163, 1981/1982; SILVA-FILHO, P. V.; BRANDO, M. Plantas medicinais de uso popular coletadas e comercializadas na região metropolitana de Belo Horizonte. Daphne, v. 2, n. 2, p. 3- 53, 1992; RODRIGUES, V. E. G.; CARVALHO, D. A. Levantamento etnobotanico de plantas medicinais no domínio Cerrado na região do Alto Rio Grande - Minas Gerais. Cienc. Agrotec., v. 25, n. 1, p. 102 - 123, 2001), as anti-inflammatory agents (LIMA, J. C. S.; MARTINS, D. T. Screening farmacológico de plantas medicinais utilizadas popularmente como antiinflamatórias. In: SIMPÓSIO DE PLANTAS MEDICINAIS DO BRASIL, 14, Florianópolis. Resumos... Florianópolis Universidade Federal do Paraná, 1996. p. 89), for treating diabetes and for weight loss diets (GRANDI, T. M. S.; LIMA-FILHO, F. M.; FERREIRA, S. M. A. Levantamento das plantas medicinais de Grão Mogol. Oréades, v. 8 (14/15), p. 116 - 125, 1981/1982); the roots are used for treating rheumatism, as stomatic and antihypertensive agents (GRANDI, T. M. S.; LIMA-FILHO, F. M.; FERREIRA, S. M. A. Levantamento das plantas medicinais de Grão Mogol. Oréades, v. 8 (14/15), p. 116 - 125, 1981/1982; HIRSCHMANN, G. S.; ARIAS, A. R. A survey of medicinal plants of Minas Gerais, Brazil. J. Ethnopharmacol., v. 29, p. 15- 172, 1990); its latex and leaves are used as adstringents for treating dermatosis and liver diseases (BRITTO, K. B.; BRITTO, I. C. Plantas com atributos medicinais do Herbário da Universidade de Feira de Santana. Oréades, v. 8, n. 14/15, p. 152 - 163, 1981/1982; GOTTSBERGER, I. S. O cerrado como potencial de plantas medicinais e tóxicas. Oréades, v. 8 (14/15), p. 15 - 30, 1981/1982); its fruits are used as digestive (GAVILANES, M. L.; BRANDO, M. Frutos, folhas e razes de plantas do cerrado, suas propriedades medicinais, tendo como veículo a cachaça. Inf. Agropec., v. 16, p. 4- 44, 1992) and as food (MOURA, J.C. Farmacêutico Joaquim Corrêa de Mello um sábio botânico campineiro esquecido. Tribuna Farmacêutica, v. 16, n. 8, p. 144-148, 1948). In addition to its medicinal use, the latex of *H. speciosa* is a raw material for rubber production (GAVILANES, M. L.; BRANDO, M. Frutos, folhas e razes de plantas do cerrado, suas propriedades medicinais, tendo como veículo a cachaça. Inf. Agropec., v. 16, p. 4- 44, 1992). Cardiovascular diseases (hereinafter CVDs) are due to multiple risk factors and constitute the major morbi-mortality cause in the world's population, regardless the gender and the development level of the country at issue. In the whole world, 30% of known death causes result from CVDs, and 4.5% of all global diseases that are prevalent in developed and developing countries have hypertension as their main risk factor (WHO. World Health Organization - International Society of Hypertension Guidelines for the Management of Hypertension. Guidelines Subcommittee. J. Hypertens., v. 17, p. 151 - 183, 1999; World Health Organization/International Society of Hypertension Writing Group 2003. WHO/ISH statement on management of hypertension. J. Hypertens., v. 21, p. 1983 - 1992, 2003; KEARNEY, P. M.; WHELTON, M.; REYNOLDS, K.; MUNTNER, P.; WHELTON, P. K., HE, J. Global burden of hypertension analysis of worldwide data. Lancet, v. 365, p. 217 - 223, 2005; KAPLAN, N. M.; OPIE, L. H. Controversies in hypertension. Lancet, v. 367, p. 168 - 176, 2006; THAYER, J. F., LANE R. D. The role of vagal function in the risk for cardiovascular disease and mortality Biol. Psychol., v. 74, p. 224 - 242, 2007). Although approximately 25% of the world population (1 billion people) has been diagnosed as hypertensive, less than one-third of this population are adequately treated, which contributes for the high mortality level by hypertension (TUNSTALL-PEDOE, H.; KUULASMAA, K., MHNEN, M.; TOLONEN, H.; RUOKOKOSKI, E.; AMOUYEL, P.; Contribution of trends in survival and coronary - event rates to changes in coronary heart disease mortality 10-year results from 37 WHO MONICA Project populations. Lancet, v. 353, p. 1547 - 1557, 1999; CHOBANIAN, A. V.; BAKRIS, G. L.; BLACK, H. R.; CUSHMAN, W. C.; CREEN, L. A.; IZZO, J. L.; JONES, D. W.; MATERSON, B. J.; OPARIL, S.; WRIGHT, J. T. Jr.; ROCELLA, E. J. The seventh report of the Joint National Committee on Prevention, Detection, Evaluation, and Treatment of High Blood Pressure. JAMA, v. 289, n. 19, p. 256- 2572, 2003; WHO World Health Organization/International Society of Hypertension Writing Group 2003. WHO/ISH statement on management of hypertension. J. Hypertens., v. 21, p. 1983 - 1992, 2003; KEARNEY, P. M.; WHELTON, M.; REYNOLDS, K.; MUNTNER, P.; WHELTON, P. K., HE, J. Global burden of hypertensionanalysis of worldwide data. Lancet, v. 365, p. 217 - 223, 2005; KAPLAN, N. M.; OPIE, L. H. Controversies in hypertension. Lancet, v. 367, p. 168 - 176, 2006). Treating the aforesaid disease should be individualized in addition to including educational strategies, changes in life habits and pharmacotherapeutics. Several factors should be considered for an adequate pharmacotherapeutic selection for controlling hypertension, namely: age, renal and hepatic functions, as well as concomitant diseases. It is well known that diabetes mellitus and chronic renal insufficiency are morbidity cofactors. These pathologies should be jointly treated in order to reduce mortality risks (WHO. World Health Organization - International Society of Hypertension Guidelines for the Management of Hypertension. Guidelines Subcommittee. J. Hypertens., v. 17, p. 151 - 183, 1999; CHOBANIAN, A. V.; BAKRIS, G. L.; BLACK, H. R.; CUSHMAN, W. C.; CREEN, L. A.; IZZO, J. L.; JONES, D. W.; MATERSON, B. J.; OPARIL, S.; WRIGHT, J. T. Jr.; ROCELLA, E. J. The seventh report of the Joint National Committee on Prevention, Detection, Evaluation, and Treatment of High Blood Pressure. JAMA, v. 289, n. 19, p. 256- 2572, 2003; WHO...2003; BRASIL Ministério da Saúde. Agência Nacional de Vigilância Sanitária. Resoluçäo da Diretoria Colegiada (RDC) nº 48 de 16 de maro de 2004. Dispõe sobre o registro de Fitoterápicos. Diário oficial da União, 2004 KAPLAN, N. M.; OPIE, L. H. Controversies in hypertension. Lancet, v. 367, p. 168 - 176, 2006; THAYER, J. F., LANE R. D. The role of vagal function in the risk for cardiovascular disease and mortality Biol. Psychol., v. 74, p. 224 - 242, 2007).

The renin-angiotensin system (hereinafter RAS) mediates several effects on the cardiovascular system, including blood pressure homeostasis and maintenance of the hydric-electrostatic balance. ACE, also called Kininase II, is an enzyme responsible for the break of dipeptides in the C-terminal portion of angiotensin I, bradykinin and several other small peptides with no proline as the penultimate amino acid in the C-Terminal (Skidgel R.A. e Erdos E. The Renin Angiotensin System. Raven Press Ltda, v. 1, 10.1-10.10, 1993). The said enzyme is connected to the endothelial, epithelial cells membrane and in soluble form in the blood and other corporeal fluids (ibid). The changing phase of inactive angiotensin I peptide into active angiotensin II peptide is crucial for the RAS role in regulating cardiovascular function. Angiotensin II is a potent vasoconstrictor, acting directly on vascular smooth muscle (Folkow B. et al., Acta Physiol. Scand. 53, 99-104, 1961). Additionally, angiotensin II also stimulates the sympathetic nervous system (peripheral and central), increasing vascular tonus. It also induces sodium retention, through aldosterone release and renal vasoconstriction, and liquid retention by the effect of the antidiuretic hormone (Biron P. et al., J. Clin. Invest. 60, 338-347, 1961; Padfield P.L. e Morton J.J. J. Clin. Endocrinol. 74, 251-259, 1977). At cellular level, angiotensin II induces migration, proliferation and hypertrophy of cells (Bell L. e Madri J. Am. J. Pathol. 137, 7-12, 1990; Itoh H. J. Clin. Invest. 91, 2268-2274, 1993).

Developing ACE inhibitors as antihypertensive agents has first occurred based on an academic research carried out by Professor Sérgio Ferreiras group in Brazil by which jararaca (Bothrops jararaca) venom has proved to possess components that intensify responses to bradykinin, at first named bradykinin potentiating factor. The isolation of such peptides and the study of their mechanisms of action by Brazilian and American research groups have led to the conclusion that aforesaid responses are ACE inhibitors, which have turned out to be prototypes of the angiotensin converting enzyme inhibitors (ACEI) class. Captopril was the first nonpeptide ACEI to be synthesized and released for clinical use, which was then followed by several others now also in use (FERREIRA ROCHA E SILVA, M. Potentiation of bradykinin and eledoisin by BPF (bradykinin potentiating factor) from Bothrops jararaca venom. Experientia, v. 21, n. 6, p. 347 - 349, 1965; FERREIRA, S. H.; BARTELT, D. C.; GREENE, L. J. Isolation of bradykinin-potentiating peptides from Bothrops jararaca venom. Biochemistry, v. 9, n. 13, p. 2583 - 2593, 1970; CUSHMAN CHEUNG, H. S.; SABO, E. F.; ONDETTI, M. A. Design of potent competitive inhibitors of angiotensin-converting enzyme. Carboxyalkanoyl and mercaptoalkanoyl amino acids. Biochemistry, v. 16, p. 5484 - 5491, 1977; PETRILLO, E. W. Jr.; ONDETTI, M. A. Angiotensin converting enzyme inhibitors: medicinal chemistry and biological actions. Med. Res. Rev., v. 2, p. 1 - 41, 1982; FERREIRA University discoveries and intellectual property rightsfrom Bothrops jararaca bradykinin potentiating peptides to angiotensin converting enzyme inhibitors. Braz. J. Med. Biol. Res., v. 27, n. 8, p. 1693 - 1698, 1994; CUSHMAN, ONDETTI, M. A. Design of angiotensin converting enzyme inhibitors. Nat. Med., v. 5, n. 1, p. 111- 1112, 1999; LIMA, D. P. Synthesis of angiotensin-converting enzyme (AEC) inhibitors as important class of antihypertensive drugs. Quím. Nova, 22(3), p. 375 - 381, 1999).

Synthetic ACEIs are vastly used and spread and are clinically efficient in treating hypertension, congestive cardiac insufficiency, angina and in preventing sudden death (The Task Force on ACE-inhibitors of the European Society of Cardiology. Eur. Heart J. 25, 1454-1470, 2004). However, several side effects have been observed in the long run, including cough, taste disturbances, hyperpotassemia and skin fissures (CLELAND, J. G. F; SWEDBERG, K.; POOLE-WILSON, P. A. Successes and failure of current treatment of heart failure. Lancet, v. 352, p. SI1- SI28, 1998). Research on new ACEIs derived from natural sources may be a future alternative for controlling hypertension with reduced side effects.

Based on the capacity of ACE to be compatible with various substrates, it is possible to inhibit such enzyme using in vitro models and thus setting protocols for quantifying inhibition percentage, viewing to screen potential inhibitors from plant extracts and isolated natural products.

Despite being exhaustively referred to in ethnobotanic surveys, only few biological activities of Hancornia speciosa have been assessed. ACE inhibiting activity for ethanolic extract from the leaves of the said species has been referred to by Serra et al. (SERRA, C. P.; CORTES, S. F.; LOMBARDI, J. A.; BRAGA DE OLIVEIRA, A.; BRAGA, F. C. Validation of a colorimetric assay for the in vitro screening of inhibitors of angiotensin-converting enzyme (ACE) from plant extracts. Phytomedicine, v. 12, n. 6 - 7, 2005). These authors assessed several species of the Brazilian flora as for their ACE inhibiting potential. Among active species, H. speciosa inhibited said enzyme in 50.1% of the cases using the method described by Elbl; Wagner (ELBL, G.; WAGNER, H. A new method for the in vitro screening of inhibitors of angiotensin -converting enzyme (ACE), using the chromophore- and fluorephere- labelled substrate dansyltriglycine. Planta Med., v. 57, p. 137 - 141, 1991), and modified by Braga et al. (BRAGA. F. C., WAGNER, H., LOMBARDI, J. A. OLIVERIA, A. B. Screening the Brazilian flora for antihypertensive plant species for in vitro angiotensin I - converting enzyme inhibitions activity. Phytomedicine, v. 7, n. 3, p. 245 - 250, 2000), using a 0.33 mg/ml concentration, which revealed an inhibition percentage of 45.7% when assessed by using the colorimetric method approved by the group (SERRA, C.P.; CORTES, S. F.; LOMBARDI, J. A.; BRAGA DE OLIVEIRA, A.; BRAGA, F. C. Validation of a colorimetric assay for the in vitro screening of inhibitors of angiotensin-converting enzyme (ACE) from plant extracts. Phytomedicine, v. 12, n. 6 - 7, 2005), in the concentration of 0,1 mg/ml. Ferreira et al. (FERREIRA, H. C.; SERRA, C. P.; ENDRINGER, D. C.; LEMOS, V. S.; BRAGA, F. C;CORTES, S. F. Endothelium-dependent vasodilation induced by Hancornia speciosa in rat superior mesenteric artery. Phytomedicine, v. 14, p. 473 - 478, 2007, FERREIRA, H. C.; SERRA, C. P.; LEMOS, V. S.; BRAGA, F. C.; CORTES, S. F. Nitric oxide-dependent vasodilatation by ethanolic extract of Hancornia speciosa via phosphatidyl-inositol 3-kinase. J. Ethnopharmacol. , v. 109, n. 1, p. 161 - 164, 2007b). These authors have reported an endothelium-dependent vasodilating activity, by way of nitric oxid, for the ethanolic extract from the leaves of said species.

Reports on the chemical composition of barks and leaves of H. speciosa were recently published in conference abstracts (RODRIGUES, C. M.; BRITO, A. R. M. S.; HIRUMA-LIMA, C. A.; VILEGAS; W. Constituintes químicos das cascas de Hancornia speciosa Gom.(Apocynaceae) In: REUNIÃO ANUAL DA SOCIEDADE BRASILEIRA DE QUMICA, 29, Águas de Lindóia. Resumos... São Paulo, 2006; SANTOS, A. F.; RIBEIRO, C. A.; POLESE, L.; ERNANDES, J. R.; KESSERLINGH, S. M.; NONATO, R. V. Determinação de parâmetros de validação de métodos cromatográficos para análise de 5-hidroximetilfurfural e açúcares em amostras de processo de produção de polímero biodegradável. Ecl. Qum., v. 31, n. 1, p. 13 - 20, 2006) and paper (RODRIGUES; RINALDO, D.; DOS SANTOS, L. C.; MONTORO, P.; PIACENTE, S.; PIZZA, C.; HIRUMA-LIMA, C. A.; SOUZA BRITO, A. R. M.; VILEGAS, W. Metabolic fingerprinting using direct flow injection electrospray ionization tandem mass spectrometry for the characterization of proanthocyanidins from the barks of Hancornia speciosa. Rapid Commun. Mass Spectrom., v. 21, p. 1907 - 1914, 2007). From the infusion of the barks of H. speciosa, was isolated the gaclic acid, 4-hidroxi-3,5-dimetoxibenzoic (syringic acid), 3-O-caffeoylquinic , 5-0-caffeoylquinic, also cis-methyl chlorogenate, trans-methyl chlorogenate , 2,7-diidroxixantona e 2,7-dimetoxixantona (RODRIGUES, C. M.; BRITO, A. R. M. S.; HIRUMA-LIMA, C. A.; VILEGAS; W. Constituintes químicos das cascas de Hancornia speciosa Gom.(Apocynaceae) In:REUNIÃO ANUAL DA SOCIEDADE BRASILEIRA DE QUÍMICA, 29, Águas de Lindóia. Resumos... São Paulo, 2006).

A dry residue analysis of the H. speciosa leaf hydrodistillate by chromatography coupled with mass spectrometer has allowed identifying the following substancestrans-linalool oxide, cis-linalool oxide, α-terpinol, linalool, geraniol, methyl-anthranilate, eugenol and (E)-isoeugenol (SANTOS, A. F.; RIBEIRO, C. A.; POLESE, L.; ERNANDES, J. R.; KESSERLINGH, S. M.; NONATO, R. V. Determinação de parâmetros de validação de métodos cromatográficos para análise de 5-hidroximetilfurfural e açúcares em amostras de processo de produção de polímero biodegradável. Ecl. Quím., v. 31, n. 1, p. 13 - 20, 2006).

A bark infusion of H. speciosa has been assessed by mass spectrometry aiming at identifying procyanidin. The following procyanidins have been detected: epicatechin -(4β→8)-catechin, epicatechin-(4β→6)-catechin) and epicatechin -(2β→7; 4β→8)- epicatechin (RODRIGUES ; RINALDO, D.; DOS SANTOS, L. C.; MONTORO, P.; PIACENTE, S.; PIZZA, C.; HIRUMA-LIMA, C. A.; SOUZA BRITO, A. R. M.; VILEGAS, W. Metabolic fingerprinting using direct flow injection electrospray ionization tandem mass spectrometry for the characterization of proanthocyanidins from the barks of Hancornia speciosa. Rapid Commun. Mass Spectrom., v. 21, p. 1907 - 1914, 2007).

Pharmaceutical compositions derived from aminocyclitols and their individual anomers are described in the patent US4590179. The following elements are also included in the said invention: process for the obtention of aminocyclitol derivatives and their individual anomers; compositions containing aminocyclitol derivatives or anomers; and methods for using such derivatives and anomers for treating diabetes and hyperlipidemy.

Patent US5428066 describes a method for treating diseases associated with high blood sugar levels, including a chiro-inositol dietary supplement. Illnesses usually associated with insulin resistance, such as hypertension, lactic acidosis, obesity and coronary artery diseases are appropriately treated with chiro-inositol in order to reach a normal metabolic level.

Pinitol and derivatives, as well as their pharmaceutical compositions used to make free fatty acids to decline or for treating conditions associated with insulin resistance, such as diabetes mellitus and its chronic complications, obesity, hyperlipidemy and atherosclerosis, hypertension, cardiovascular diseases, AIDS, cancer, sepsis, burn traumas, malnutrition, stress, lupus and other autoimmune diseases, endocrine diseases, hyperuricemy, polycystic ovary syndrome and complications resulting from athletic activity described by patent US5827896.
Some patents describe compounds with agonistic or antagonistic properties of inositol phosphoglycan. For instance, the patent US6939857 describes compounds based on substituted cyclitols, such as chiro-inositol, derived from pinitol (3-O-methyl-chiro-inositol) and their uses.

Some studies describe a new method for screening plant extracts that are potential inhibitors of ACE. The ACE inhibiting activity of the *Hancornia speciosa* extract was previously reported by the research group requiring the present patent (SERRA C. P.; CORTES, S. F.; LOMBARDI, J. A.; BRAGA DE OLIVEIRA, A.; BRAGA, F. C. Validation of a colorimetric assay for the in vitro screening of inhibitors of angiotensin-converting enzyme (ACE) from plant extracts. Phytomedicine, v. 12, n. 6 - 7, 2005.). However, the said report is different from the present invention as it only refers to an in vitro assessment of the plant crude extract rather than its standardized fraction, which is obtained by means of the process reported herein, whose constitution is rich in enantiomer L-(+)-bornesitol, rutin and quinic acid. (C.P. Serra, S.F. COrtes, J.A. Lombardi, A. Braga de Oliveira, F.C. Braga. Validation of a colorimetric assay for the in vitro screening of inhibitors. Phytomedicine, v. 12, n. 6 - 7, 2005).

Another study by the research group requiring the present patent (FERREIRA, H. C.; SERRA, C. P.; ENDRINGER, D. C.; LEMOS, V. S.; BRAGA, F. C;CORTES, S. F. Endothelium-dependent vasodilation induced by Hancornia speciosa in rat superior mesenteric artery. Phytomedicine, v. 14, p. 473 - 478, 2007) describes the vasodilating effect of *Hancornia speciosa* ethanolic extract on rat aortic preparations. Similarly to the aforementioned experiment, this study was carried out with crude extract but not with the standardized fraction rich in enantiomer L-(+)-bornesitol, rutin and quinic acid, which is the object of the present invention patent required herein.(HERICK C. FERREIRA, CARLA P. SERRA, VIRGINIA S. LEMOS, FERNÃO C. BRAGA, STEYNER F. CORTES).

Although the state of the art includes several pharmaceutical compositions for treating cardiovascular disturbances in general, as well as their obtention processes, none of such compositions proposes their production process or any pharmaceutical compositions of the standardized extract of *Hancornia speciosa Gomes* leaves, as well as of their standardized fraction rich in bornesitol, rutin and quinic acid, with ACE inhibiting, vasodilating, antihypertensive and antioxidant activities.

The composition of the present invention is characterized by the use of a pharmaceutically acceptable excipient mixture combined with a standardized fraction of *Hancornia speciosa* rich in bornesitol, rutin, quinic acid and other polyols and polyfenols, as well as their natural or synthetic analogues. Examples of excipient include water, saline solution, buffer solutions containing phosphate, Ringer solution, dextrose solution, Hank solution and biocompatible saline solutions with or without polyethylene glycol. Nonaqueous vehicles, such as fixed oils, sesame oil, ethyl-oleate or triglycerides can also be used. Compositions containing one excipient or a combination thereof can be prepared.

Excipients may contain small amounts of additives, such as substances that increase substance isotonicity and chemical stability or buffers. Examples of buffers include phosphate buffer, bicarbonate buffer, Tris buffer, while preservative examples include thimerosal, m- or o-cresol, formalin and benzyl alcohol. Standardized compositions may be liquid or solid. Therefore, excipient may include dextrose, albumin of human serum, preservatives, etc. in a nonliquid formulation to which water of saline solution may be added before delivery.

The aforesaid compositions may be administered by inhalation or by intramuscular, intravenous, subcutaneous and oral routes, as well as by implanted or injected devices, although they should preferably delivered orally.

### Disclosing the invention:

The present invention is characterized by standardized extract obtained from the leaves of *Hancornia speciosa Gomes* (EHS), usually called mangaba, as well as a fraction thereof rich in bornesitol, rutin and quinic acid, with ACE inhibiting, vasodilating, antihypertensive and antioxidant activities. Particularly, the said invention refers to a fraction thereof rich in enantiomer L-(+)-bornesitol, rutin and quinic acid from the leaves of the aforesaid vegetal species containing activities that are ACE inhibiting, vasodilating, anti-hypertensive and antioxidant.

The said standardized fraction and substances described in the present invention were assayed and presented ACE inhibiting activity, vasodilating activity dependent and independent of vascular endothelium, and hypotensive activity that were superior to those of the extract. The plant extract, fraction and substances were also able to reduce arterial pressure in hypertensive animal models.

The present invention is the first one to report the interaction between cyclitol L-(+)-bornesitol, rutin flavonoid, quinic acid and other polyols and flavonoids for treating pathologies related to the cardiovascular system.

The present invention also comprises the use of L-(+)-bornesitol, rutin, quinic acid and other polyols and polyfenols, as well as their natural and synthetic analogues and pharmaceutical compositions containing pharmaceutically and pharmacologically acceptable excipients for treating cardiovascular system pathologies.

Therefore, in addition to the novel biological activities reported for the standardized extract and fraction of the said plant, as well as for L-(+)-bornesitol and rutin chemical markers, the process for obtaining the said fraction and its standardization, in chemical, quantitative and biological activity terms, is equally a novelty.

The present invention can be better understood by the following nonlimiting examples.

### Example 1: Obtaining a standardized fraction of Hancornia speciosa

The non washed leaves of *Hancornia speciosa* are directly submitted to stabilization and dried in an oven with circulating air at 40 °C for 72h. The dried vegetal material is pulverized in a cutting mill and percolated up to exhaustion with ethanol at 96°GL. Subsequently, the ethanolic extract is concentrated in a rotary evaporator at 50°C under reduced pressure and the resulting residue was kept in a dessicator, under vacuum, for eliminating the residual solvent for at least 48h.

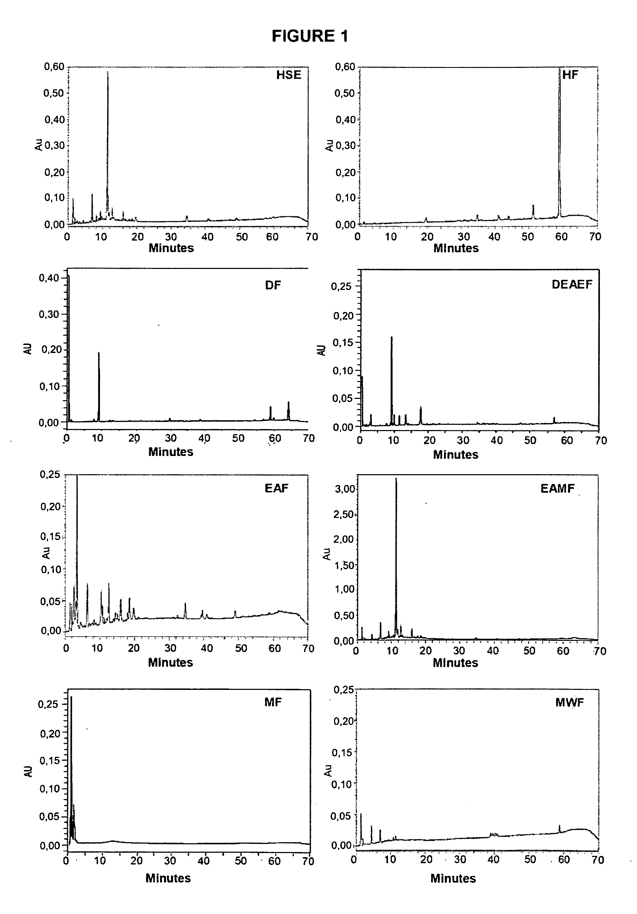

The crude extract fractioning is carried out in a silica-gel open column (0.2 mm to 5mm mesh) with a 1:12 extract/adsorbent ratio.The eluotropic series used comprises the following: n-hexane, dichloromethane, dichloromethane/ethyl acetate (1:1), ethyl acetate, ethyl acetate/methanol (1:1), methanol; methanol/water (1:1) and aqueous solution of acetic acid at 5 %. The reference chromatographic profiles, obtained by reversed-phase high performance liquid chromatography (RP-HPLC), can be found in Figure 1. The standardized fraction, enriched in cyclitols and flavonoids, corresponds to the eluate obtained with ethyl acetate/methanol (1:1), yielding 45% w/w (weight/weight) in relation to the original extract.

Under controlled conditions, the standardized fraction enriched in cyclitols and flavonoids contains 17% w/w of L-(+)-bornesitol and 10% w/w of rutin, along with canferol-3-*O*-rutinoside, 5-*O*-caffeoylquinic, *trans*-4-hydroxycinamic and *cis*-4-hydroxycinnamic acids, according to the chemical fingerprint obtained by high performance liquid chromatography with diode array detector (HPLC-DAD).

### Example 2: ACE inhibiting activity of the standardized fraction and crude extract

**The standardized fraction** enriched in cyclitols and flavonoids **produced 95 ± 13% of ACE inhibition** when assessed at the concentration of 100 µg/mL, using the colorimetric assay described by Serra et. al. (SERRA, C. P.; CORTES, S. F.; LOMBARDI, J. A.; BRAGA DE OLIVEIRA, A.; BRAGA, F. C. Validation of a colorimetric assay for the in vitro screening of inhibitors of angiotensin-converting enzyme (ACE) from plant extracts. Phytomedicine, v. 12, n. 6 - 7, 2005), whereas the **crude extract resulted in a 59 ± 13 %** inhibition in the same assay.

The above mentioned standardization procedure encompass optimization of the extraction and fractioning processes, as well as on the ACE inhibiting activity assayed for the major chemical markers of the fraction: L-(+)-bornesitol (IC₅₀ = 41.4 ± 9.6 µM) and rutin (IC₅₀ = 453.9 ± 78.4 µM).

### Example 3: Vasodilating Effect

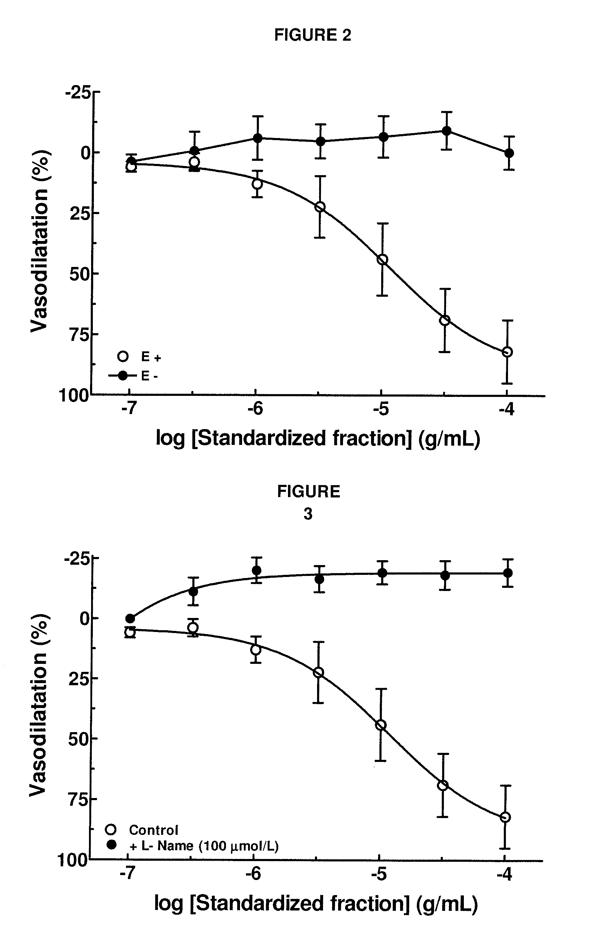

The EHS standardized fraction produced vasodilating effect dependent on the presence of intact vascular endothelium in rat aorta precontracted with phenylephrine (IC₅₀ = 11.5 ± 1.0 µg/mL), as shown in figure 2. This outcome shows that the standardized fraction is able to significantly activate only arteries with integral endothelial layer. Such effect on rat aorta generally occurs due to release of nitric oxide or cyclooxygenase derivatives.

### Example 4: Inhibition of the Vasodilating Effect

Based on the present example, it is possible to conclude that the inhibition of nitric oxide production induced by L-NAME (100µM) in aorta rings precontracted with phenylephrine has abolished the vasodilating effect induced by the **standardized fraction,** as shown in figure 3. Such a result proves that this fraction induces its vasodilating effect by means of a mechanism dependent on the vascular endothelial production of nitric oxide. Therefore, from the clinical viewpoint, such fraction may have beneficial effects connected with antihypertensive, antioxidant and cellular proliferation inhibiting actions in cases of restenosis associated with nitric oxide.

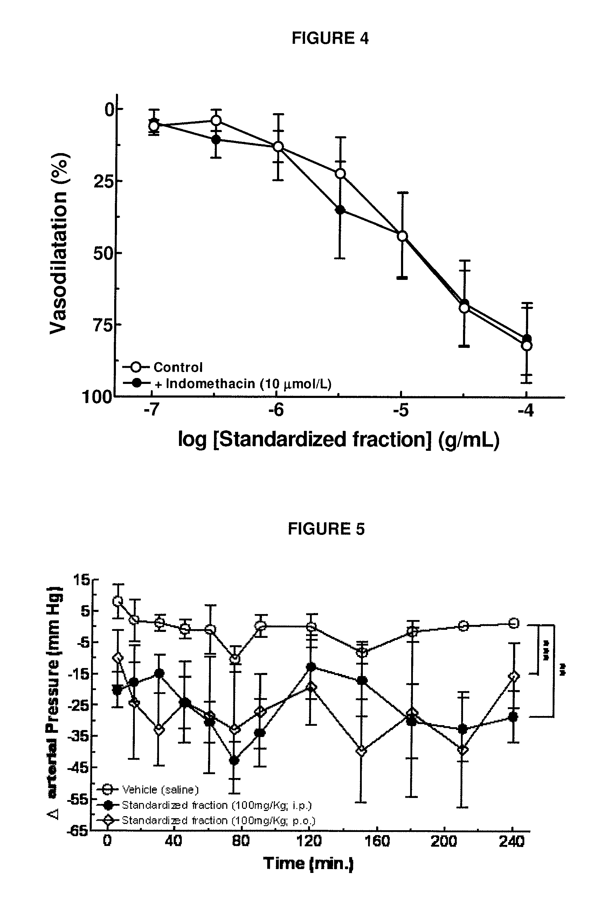

Cyclooxygenase derivatives, such as prostacyclin, are involved in endothelium-dependent vasodilation in several vascular beds. In order to verify whether any cyclooxygenase derivative was involved in the vasodilating effect induced by the **standardized fraction,** indomethacin (10 µM) was delivered and no alteration in vasodilating effect on the rat aorta precontracted with phenylephrine was noticed (Figure 4). This outcome shows that cyclooxygenase derivatives are not likely to take part in vasodilation of our fraction.

### Example 5: Reduced systolic pressure in normotensive mice

**The standardized fraction** (10mg/Kg) significantly reduced the systolic pressure measured in the caudal vessel in normotensive mice when delivered orally, p. o, (*** P < 0.001 *versus* vehicle) and by intraperitoneal route, p.i., (** P < 0.01 *versus* vehicle) for more than 4 hours, as illustrated in figure 5. Such results show that this fraction has also a hypotensive effect, in addition to the vasodilating effect.

### Example 6: Reduced systolic pressure in hypertensive mice

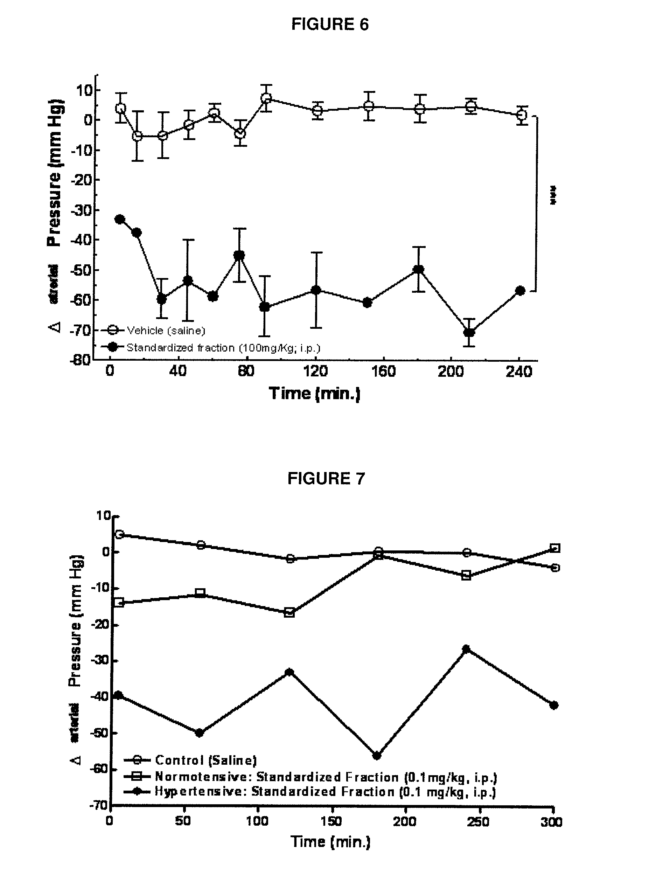

The standardized fraction (100 mg/Kg) reduced dramatically the systolic pressure in hypertensive mice (Doca-sal) when delivered via intraperitoneal route (*** P < 0.001 versus vehicle), as can be seen in figure 6. The hypotensive effect was significantly stronger (P < 0.05) on hypertensive animals than that observed for normotensive animals. This suggests that the said fraction has an antihypertensive effect that can be used in lower doses in hypertensive animals.

### Example 7: Reduced systolic pressure in normotensive and hypertensive mice

As for normotensive mice, the standardized fraction (0.1 mg/Kg), delivered intraperitoneally, reduced slightly-about 10 mmHg - the arterial pressure for a short time span, approximately 2h (Figure 7). However, when delivered to hypertensive mice (Doca-sal), the same dose of standardized fraction induced a strong systolic pressure reduction - approximately 40 mmHg - whose effect lasted for more than 5h (Figure 7). These results suggest that hypertensive individuals are more sensitive to the standardized fraction's antihypertensive action and that small doses of said fraction may be used for treating hypertension with no significant alteration in the cardiovascular function in normotensive individuals.

### Example 8 - Comparison of the hypotensive effect of the standardized fraction and an ACE inhibitor

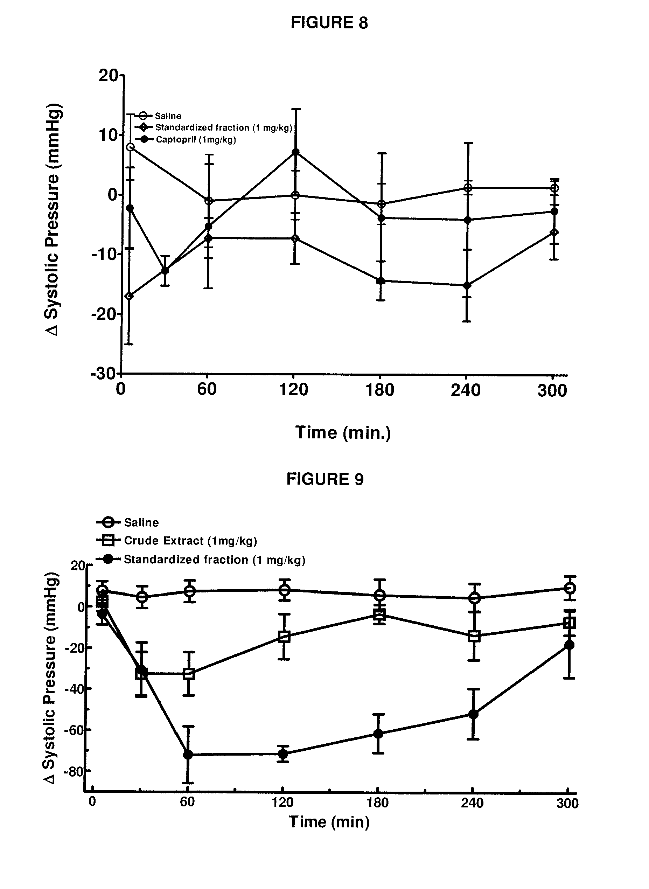

In normotensive mices the standardized fraction (1 mg/kg) delivered orally, significantly reduced the systolic arterial pressure (Figure 8). Captopril (1 mg/kg), a standard ACE inhibitor, also delivered orally, showed a significant hypotensive effect, but of low intensity and short duration (Figure 9). The comparison of these effects shows that the standardized fraction of *H. speciosa* presents a hypotensive effect more intense and of longer duration than the standard ACE inhibitor.

### Example 9 - Comparison of the antihypertensive effect of the standardized fraction and the crude ethanolic extract of leaves of Hancornia speciosa

In hypertensive mices the standardized fraction (1 mg/Kg) delivered orally, highly reduced the systolic pressure (Figure 9). The maximum reduction of systolic pressure obtained with the standardized fraction was equal to 55.5 ± 8.9 mmHg and the duration of the antihypertensive effect was greater than 4 hours. The crude extract of Hancornia speciosa (1 mg/Kg) delivered orally, also significantly reduced systolic pressure of hypertensive mices, however, this effect was significantly lower than those observed with standard fraction, in the same dose (Figure 9). The maximum reduction of systolic pressure obtained with the crude extract was equal to 19.3 ± 8.4 mmHg and the duration of the effect was less than 2 hours. These examples clearly show that the standardized fraction is more potent and the duration of its antihypertensive effect is substantially higher than observed with the crude extract. Thus, it is evident the advantage of the therapeutic use of this fraction with respect to crude ethanol extract of leaves of *H. speciosa.*

### Example 10 - Effect of the standardized fraction in plasma nitrite level in normotensive mice

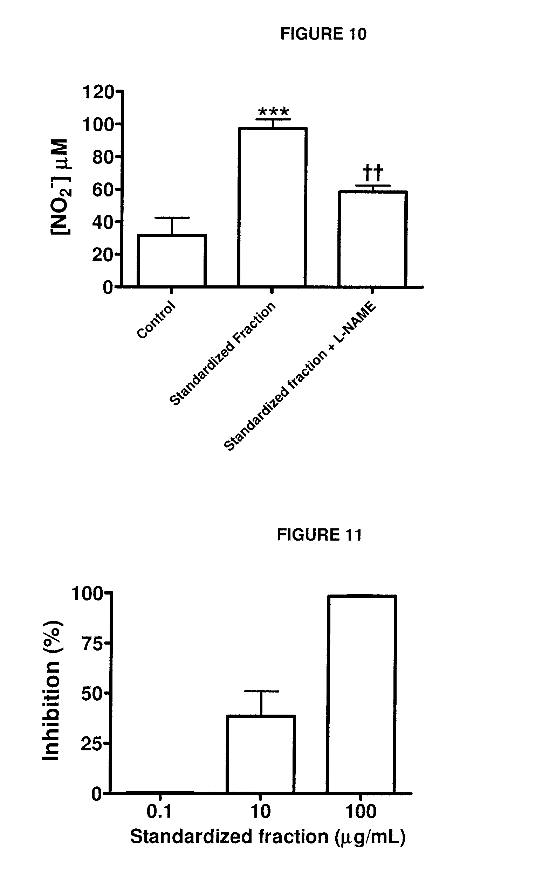

The standardized fraction of *H. speciosa* (1 mg/kg) delivered orally significantly increased the level of nitrite in plasma samples taken 1 hour after its delivered (Figure 10). The nonselective nitric oxide synthase, L-NAME (20 mg/kg) significantly inhibited this increase (Figure 10). As nitrites resulting from the degradation of nitric oxide, this result strongly suggests that the standardized fraction increases the production or bioavailability of nitric oxide. Thus, at least part of the hypotensive effect/anti-hypertensive of the standardized fraction must pass by the nitric oxide increase in the cardiovascular system.

### Example 11 - Antioxidant activity of the standardized fraction of Hancornia speciosa

The antioxidant activity of different concentrations of the standardized fraction was analyzed by chemiluminescence using luminol by the reaction between xanthine and xanthine oxidase. As illustrated in Figure 11, it can be seen that the fraction has a standard concentration-dependent antioxidant effect. This effect may contribute to the activity hypotensive/antihypertensive of this fraction, since free radicals have vasoconstrictor activity and reduce endothelial-dependent vasodilation function.

### Example 12 - Antihypertensive Activity of the standardized fraction of Hancornia speciosa delivered at a dose of 0.1 mg/kg, in drink water, for 14 days

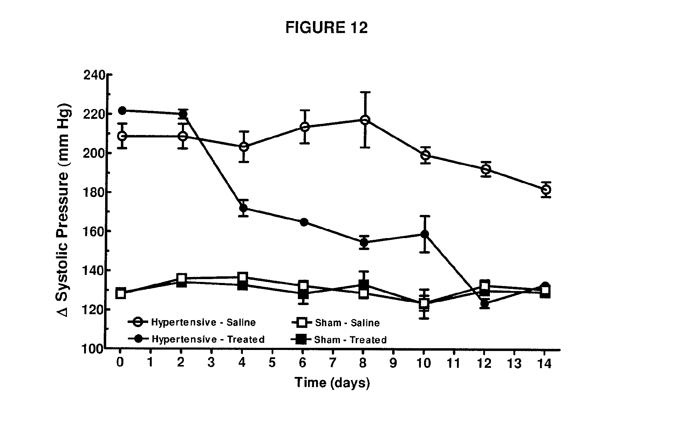

In hypertensive mices standardized fraction of *H. speciosa,* at a dose of 0.1 mg/kg, highly reduced the systolic pressure during the 14 days of the treatment (Figure 12), bringing the SBP to the same level of normotensive animals. In normotensive animals (Sham) the standardized fraction, at a dose of 0.1 mg/kg, did not affect SBP (Figure 12). These results demonstrate that the standardized fraction at low doses has antihypertensive activity during the treatment of 14 days. Treatment of normotensive mices with this fraction, during the same period of time and dose, indicating the absence of hypotensive activity.

### List of figures:

**Figure 1** - Chromatograms - obtained by RP-HPLC for the crude ethanolic extract of *Hancornia speciosa* leaves (HSE) and resulting fractions [HF, hexanic fraction; DF, dichloromethane fraction; DEAF, dichloromethane / ethyl acetate fraction (1:1); EAF, ethyl acetate fraction; EAMF, ethyl acetate / methanol fraction (1:1); MF, methanolic fraction; MWF, methanol / water fraction (1:1)]. Chromatographic conditions: gradient elution of phosphoric acid 0.1 % (A) and acetonitrile + phosphoric acid 0.1% (B) (5% B → 95% B in 60 min, followed by 5min of isocratic elution); temperature of 40 ºC; UV₂₁₀ₙₘ detection; flow rate of 1 mL/min.
**Figure 2** - Vasodilating effect dependent on concentration of *Hancornia speciosa* of the standardized fraction in the absence and presence of an intact vascular endothelium in mice aorta precontracted with phenylephrine.
**Figure 3** - Vasodilating effect induced by *Hancornia speciosa* standardized fraction in the absence and presence of an inhibitor of nitric oxide production, L-NAME (100 µM), in aorta rings precontracted with phenylephrine.
**Figure 4** - Vasodilating effect induced by *Hancornia speciosa* standardized fraction in the absence and presence of an inhibitor of cyclooxygenase, indomethacin (10 µM), in aorta rings precontracted with phenylephrine.
**Figure 5** - Hypotensive effect (mmHg) of *Hancornia speciosa* standardized fraction (100 mg/Kg) in normotensive mice delivered orally and intraperitoneally.
**Figure 6** - Hypotensive effect (mmHg) of *Hancornia speciosa* standardized fraction (100 mg/Kg) in hypertensive mice delivered intraperitoneally.
**Figure 7** - Hypotensive effect (mmHg) of *Hancornia speciosa* standardized fraction (0.1 mg/Kg) in normotensive and hypertensive mice delivered intraperitoneally.
**Figure 8** - Hypotensive effect (mmHg) of *Hancornia speciosa* standardized fraction (1 mg/Kg) and captropil (1 mg/Kg) in normotensive mice delivered orally.
**Figure 9** - Antihypertensive effect (mmHg) of *Hancornia speciosa* standardized fraction (1 mg/Kg) and the crude extract (1 mg/Kg) in hypertensive mice delivered orally.
**Figure 10** - Standardized fraction effect (1 mg/Kg), delivered orally, in the plasma nitrite concentration in normotensive mice. The animal's plasma was obtained 1 h after the fraction delivered.
**Figure 11** - Antioxidant effect of the *H. speciosa* standardized fraction observed in the reaction between xanthine and xanthine oxidase.
**Figure 12** - Antihypertensive effect of the *Hancornia speciosa* standardized fraction delivered orally, at a dose of 0.1 mg/Kg, diluted in the drink water of the hypertensive and normotensive mices.

## Claims

1. **STANDARDIZED EXTRACT AND FRACTION OF *HANCORNIA SPECIOSA* LEAVES, characterized by** comprising a standardized fraction enriched in cyclitols e flavonoids, which contains from 15 to 25% w/w of L-(+)-bornesitol and 7 to 15% w/w of rutin, in addition to canferol-3-*O*-rutinoside and 5-*O*-caffeoylquinic, *trans-4-*hydroxycinamic and *cis*-4-hydroxycinnamic acids.

2. **STANDARDIZED EXTRACT AND FRACTION OF *HANCORNIA SPECIOSA* LEAVES AND PRODUCT,** according to claim 1, **characterized by** comprising the standardized based on the optimization of extraction and fractioning processes, as well as on the ACE inhibiting activity of the major chemical makers present in the fraction: L-(+)-bornesitol and rutin.

3. **STANDARDIZED EXTRACT AND FRACTION OF *HANCORNIA SPECIOSA* LEAVES AND PRODUCT,** according to claims 1 and 2, **characterized by** said standardized fraction enriched and by the characteristic chemical fingerprint obtained by high performance liquid chromatography (HPLC), similar to that selected in Figure 1 B.

4. **PHARMACEUTICAL COMPOSITION FROM STANDARDIZED EXTRACT AND FRACTION OF *HANCORNIA SPECIOSA* LEAVES,** according to claims 1 to 3, **characterized by** comprising pharmaceutically and pharmacologically acceptable inert excipients.

5. **PHARMACEUTICAL COMPOSITION FROM STANDARDIZED EXTRACT AND FRACTION OF *HANCORNIA SPECIOSA* LEAVES,** according to claim 4, **characterized by** comprising the ACE inhibiting, vasodilating, antihypertensive, and antioxidant activities.

6. **PHARMACEUTICAL COMPOSITION FROM STANDARDIZED EXTRACT AND FRACTION OF *HANCORNIA SPECIOSA* LEAVES,** according to claims 4 and 5, **characterized by** presenting ACE inhibiting activity above 90%.

7. **PHARMACEUTICAL COMPOSITION FROM STANDARDIZED EXTRACT AND FRACTION OF *HANCORNIA SPECIOSA* LEAVES,** according to claims 4 to 6, **characterized by** presenting long-lasting hypotensive effect.

8. **PHARMACEUTICAL COMPOSITION FROM STANDARDIZED EXTRACT AND FRACTION OF *HANCORNIA SPECIOSA* LEAVES,** according to claims 4 to 7, **characterized by** inducing a significant reduction of systolic pressure effect of which lasts for more than 5 h.

9. **PHARMACEUTICAL COMPOSITION FROM STANDARDIZED EXTRACT AND FRACTION OF *HANCORNIA SPECIOSA* LEAVES,** according to claims 4 to 8, **characterized by** being administered by oral, intramuscular, intravenous, intraperitoneal, subcutaneous and transdermal routes or as devices that could be implanted or injected, although preferably delivered orally.

10. **PHARMACEUTICAL COMPOSITION FROM STANDARDIZED EXTRACT AND FRACTION OF *HANCORNIA SPECIOSA* LEAVES,** according to claims 4 to 9, **characterized by** being for the manufacture of a medicament for treating cardiovascular disturbances.

11. **PHARMACEUTICAL COMPOSITION FROM STANDARDIZED EXTRACT AND FRACTION OF *HANCORNIA SPECIOSA* LEAVES,** according to claims 4 to 10, **characterized by** being for the manufacture of antioxidants medicaments.

12. **METHOD FOR TREATING CARDIOVASCULAR DISTURBANCES, characterized by** comprising the administration of the pharmaceutical composition, described in claims 4 to 11, to a person that requires the treatment.

13. **METHOD FOR THE STUDY OF THE TREATMENT OF CARDIOVASCULAR DISTURBANCES, characterizing by** comprising the administration of the composition described in claims 4 to 11.
